## Europäisches Patentamt
## European Patent Office
## Office européen des brevets

(19)

(11) Publication number: **0 072 581**

**B1**

(12) **EUROPEAN PATENT SPECIFICATION**

(45) Date of publication of patent specification: **08.04.87**

(21) Application number: **82108583.4**

(22) Date of filing: **17.09.79**

(60) Publication number of the earlier application in accordance with Art. 76 EPC: **0 009 222**

(51) Int. Cl.⁴: **C 12 Q 1/00, C 12 Q 1/32, C 12 Q 1/50, C 12 Q 1/52, C 12 Q 1/54, C 12 Q 1/58, C 12 N 9/96**

(54) **The stabilization of working reagent solutions containing enzymes, and the use of such stabilized reagents in enzyme or substrate assays.**

(30) Priority: **20.09.78 US 944192**

(43) Date of publication of application:
**23.02.83 Bulletin 83/08**

(45) Publication of the grant of the patent:
**08.04.87 Bulletin 87/15**

(84) Designated Contracting States:
**BE CH DE FR GB IT LU NL SE**

(56) References cited:
**DE-A-2 740 957**
**FR-A-1 497 240**
**FR-A-2 232 760**
**FR-A-2 298 554**
**FR-A-2 389 133**
**GB-A-1 334 467**
**US-A-4 080 263**
**US-A-4 097 336**

**Chemical Abstracts, vol. 90(1979),Abs. no. 70583**
**HANDBOOK OF ENZYME BIOTECHNOLOGY(A. Wiseman) 1975, page 8**

(73) Proprietor: **American Monitor Corporation**
**P.O.Box 68505**
**Indianapolis Indiana 46268 (US)**

(72) Inventor: **Denney, Jerry W.**
**45 Thornhurst Drive**
**Carmel Indiana (US)**

(74) Representative: **Speidel, Eberhardt**
**Postfach 1320 Waldpromenade 26**
**D-8035 Gauting (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This is a division of co-pending application number 79103451.5, filed September 17, 1979, to which priority is claimed based on United States application number 944,192 filed September 29, 1978. Subject matter relating to NADH/NADPH stabilization is being claimed in applicant's co-pending application.

Background of the invention

A particular and long-standing problem of the attempts at accurate measurement of many blood enzymes is that unfortunately they manifest no easily-perceived indication of their presence or activity; and thus in these instances their activity is coupled with reagent enzymes which are of a type or nature which do have an associated measurable change incident to their reaction.

The "coupling" of one enzyme reaction to another is an indirect yet very useful mechanism used in assay techniques; and it involves the allowing or use of a product of a first enzyme reaction to serve as a substrate or intermediate product upon which a second enzyme acts, producing yet another product, and one of the products of the second reaction is the thing whose measurement provides in effect a desired quantitation or measurement of the particular factor of interest of the first reaction.

As an illustrative example, the directly-measurable factor of a coupled enzyme reaction is a change in the spectrophotometric absorbance due to the production or consumption of the substance NADH (nicotinamide adenine dinucleotide, reduced form) or NADPH (nicotinamide adenine dinucleotide phosphate, reduced form) which is quantitatively measurable by its characteristic of absorbing strongly in the ultraviolet region of the spectrum, and thus the measured change of that absorbance is useful to give the quantitative indication of the NADH reaction involved.

An example of the indirect measurement of an enzyme, of a coupled enzyme-reaction type, is the indirect yet quantitative measurement of the very significant blood enzyme SGOT (serum glutamic-oxaloacetic transaminase). The enzyme SGOT is measured by its role in catalyzing the first of the reactions mentioned below, and a second enzyme MDH (malic dehydrogenase) is used to catalyze a reaction involving a product of the SGOT reaction; and thus these two reactions illustrate not only a vitally-significant measurement of SGOT and apparent heart damage, but illustrate also the use of one enzyme (MDH) to measure another enzyme (SGOT) in an indirect or coupled enzyme system reaction phenomenon. The SGOT reaction may be represented as follows:

$$\text{aspartic acid+alpha ketoglutaric acid} \xrightarrow[\text{pyridoxal 5'-phosphate}]{\text{SGOT}} \text{glutamic acid+oxaloacetic acid}$$

However, an indirect method of measurement is here required, because none of the participating substances for this SGOT reaction nor the products of that reaction are easily measured. Thus the measurement of the diagnostically important SGOT activity must be coupled with a second reaction which is easily measured; and commonly that second reaction is one using the enzyme MDH as a catalyst, as follows:

$$\text{oxaloacetic acid+NADH} \xrightarrow{\text{MDH}} \text{malic acid+NAD}$$

The loss in ultraviolet absorbance of NADH, incident to its conversion to NAD, is something which is easily measured; and thus this reaction, catalyzed by the MDH, provides the desired although indirect quantitation of the activity of the amount of SGOT in the blood, and thus gives the observer the vital data as to the heart damage.

Another illustration of a coupled enzyme system, using one enzyme, lactate dehydrogenase (LDH), to measure another enzyme, serum glutamic-pyruvic transaminase (SGPT), is that of the indirect assay for SGPT, vitally significant in diagnosis of liver diseases as well as diagnosis of myocardial infarction, as follows:

$$\text{alpha ketoglutaric acid+L-alanine} \xrightarrow{\text{SGPT}} \text{l-glutamate+pyruvate}$$

Similar to the SGOT example given above, none of the participating substances for this SGPT reaction nor its reaction products are easily measured, and thus a second reaction using LDH as catalyst must be coupled with the first reaction to get an easily measurable product, as follows:

$$\text{pyruvate+NADH+H}^+ \xrightarrow{\text{LDH}} \text{lactate+NAD}^+$$

The loss in ultraviolet absorbance of NADH is easily measured; and this reaction, catalyzed by LDH, provides a useful although indirect quantitation of the amount of SGPT in the blood.

2

# 0 072 581

Enzymes are also used as reagents to measure non-enzyme substances (or enzyme substrates) which are not easily detected by ordinary color-producing dye reactions and in cases where other substances are mistakenly measured by such reactions as supposedly being the substance for which the measurement was intended. More particularly, the known high specificity of enzymes is used to convert substances to be measured in the blood to a compound which can be more easily and specifically measured.

In other cases, the enzyme reagent produces an easily measurable change while converting the substance of interest in whatever is the particular reaction. The widely used hexokinase measurement of blood glucose is an example:

$$\text{glucose} + \text{ATP} \xrightarrow{\text{hexokinase}} \text{glucose-6-phosphate}$$

$$\text{glucose-6-phosphate} + \text{NAD} \xrightarrow{\text{G-6-PDH}} \text{NADH} + \text{gluconolactone-6-phosphate}$$

Thus, two enzyme reagents are used to convert glucose to gluconolactone-6-phosphate. The NADH formed absorbs ultraviolet light strongly; and the spectrophotometric absorbance at 340 nm is thus useful as a measurable determination of the glucose concentration in the blood sample used.

Because of the stability problem (short "shelf life") of enzymes in the liquid solution form in which they are used, reagent enzymes used in these tests are generally supplied in the dry form. This "dry form" storage method, although beneficial in prolonging the enzyme stability, has a great disadvantage of causing the laboratory to have to then reconstitute the enzyme reagent with water to obtain a working reagent for use in the assay. A "working reagent", more fully described below, is one which is added to the diagnostic test directly, without additional preparation. Working enzyme reagents are desirably used in these tests or assays in the form of a liquid solution, for reasons such as ease of handling and measured delivery; but in such form of liquid solution such enzyme reagents are characteristically stable for only a few hours and at most a few days, even under refrigeration. And use of enzyme reagents at the more convenient room temperature may drastically reduce stability even further.

Stability of working enzyme reagents is herein meant to refer to the retention of the utility of those enzyme reagents in performing clinical assays at some period of time (generally being about one month after preparation) in yielding clinically-useful diagnostic results.

At best, the instability of relatively expensive enzyme reagents creates economic waste, since the unused reagent must be discarded. At worst, the unstable reagent may cause a mistaken diagnostic measurement to be made; for enzyme reagents which have lost activity may cause falsely elevated or decreased measurements. Deterioration may also have the effect of altering the range of the assay, so that a severe abnormality may be reported as normal or only slightly elevated. Further, the short stability of course leads to the relatively expensive and bothersome laboratory procedure of reagent-preparation in relatively small batches. Such disadvantages of short-stability enzyme reagents have been long realized.

In spite of all this, increasing dependence upon laboratory diagnostic blood tests in the practice of medicine has caused the prior art both to use short-stability enzyme reagents in spite of these disadvantages, and to attempt to utilize various types of procedures and concepts to lengthen enzyme-stability to as long as possible, even though such attempts have generally not accomplished working reagent stability for more than a few hours or at most a day, even under refrigeration.

Enzyme instability and prior art stabilization attempts

It is known, as mentioned above, that when enzymes are in aqueous solutions, they rapidly lose their activity. Thus, reagents so prepared "decay" because of loss of enzyme activity, and after a short period of time, they cannot and should not be used due to such activity loss; for a loss of enzyme activity, of such significant amount, often occurs in as short a period of time as a few hours, even when the enzyme reagent is refrigerated.

Refrigeration of aqueous solutions of enzymes has been known by the prior art to relatively prolong stability to a certain extent. However, even under careful refrigeration, enzyme solutions generally lose substantial activity in only a few hours or at most one day.

Indeed, since all enzymes come from natural sources such as plant, animal, and microbial life, it seems that the well known property of instability of enzymes in aqueous solutions is inherently natural and vitally important in such life forms, even though the present inventive concepts achieve a result quite opposite to this apparently natural characteristic of enzymes. In nature, enzymes are used by living organisms to synthesize and catabolize virtually every compound making up the organism, as well as to control energy utilizations; and an important means which seemingly is inherently employed in nature to control such life-processes is the control of enzyme production or synthesis. If, in nature, enzymes were totally stable in the aqueous media which makes up the internal environment of all life, their level would never be reduced, only increased, and consequently control of metabolic processes would be lost.

As a result of this inherent and natural enzyme instability in an aqueous solution, considerable amount of time must be spent in preparation of fresh aqueous enzyme reagent systems known as "working reagents". A working reagent is one which is used in an "as is" condition in an enzyme assay test without

3

additional preparative steps such as adding dry, salt-suspended, or glycerol-stabilized enzymes. A further characteristic of a "working reagent" is that the ingredients of the reagent are in aqueous solution, as contrasted to being in suspension or glycol/water solution.

In addition, working reagents should be of such volume as to be conveniently and accurately added to the enzyme assay mixture in the course of performing an assay. In particular, very small volume additions are cumbersome, time-consuming, and not as likely to be accurate as are larger volumes, either in manual or automated assay methods. To minimize volume measurement error, it is desirable that each reagent addition be as large a proportion of the final volume as possible. That is, if two reagents are used, it is generally desirable that each be about one-half of the total final volume.

In addition to "working reagents" being desirable from the standpoint of efficiency due to elimination of added steps and minimization of volume error, working reagents are conducive to repeatability of ingredients from one assay to another, which repeatability may be vital to accurate assay from patient to another. The enhanced reproducibility of working reagents is due to the fact that ingredients are in solution in working reagents, rather than being dry or in suspension. Solutions by definition are uniform in concentration from one portion of the liquid to another. By contrast, suspensions are not homogeneous; and consequently, particularly if a small sample is used in each test, the sample may differ in the amount of suspended ingredient.

The raw material enzymes used by the prior art to prepare working reagents have been stabilized by drying, by suspension in glycerol solutions, and by suspension in concentrated solution (3—6 equivalents per liter) of ammonium or sodium as the sulfate salts. Although useful in stabilizing raw materials, these means are not usable in stabilizing working reagents, and the working reagents produced from such raw materials by the prior art are unstable. Dry enzymes are not easily dispensable and must go into solution prior to use, and cannot easily be used as working reagents. Enzymes in glycerol are difficult to use as a working reagent because glycerol is very viscous; and dispensing, mixing, and bubble inclusion are problems in spectrophotometric observations. Enzyme suspensions cannot be used as working reagents because the enzymes contained are not in solution, and accurate sampling is a problem. Further, the high concentrations of ammonium or sodium sulfate would likely adversely affect the properties of the enzyme assay for which the working reagent is intended.

Thus, instability seems to be natural and a fundamental property of enzymes. Although the instability property seems desirable *in vivo* in nature, the inherent instability of enzymes is indicated above as being generally undesirable in an *in vitro* use of enzymes in assay methods. Indeed, it seems that even the well-known natural and inherent instability of enzymes in aqueous solutions has been taken for granted by the prior art when employing enzymes as reagents. And refrigeration (which slows most chemical and biological processes) has been used to slow the process of decay somewhat, although to only a few hours.

The prior art acceptance (or perhaps its wide-spread recognition, acquiescence, and even resignation of or to the fact) of the unstable nature of enzymes has apparently led to a corresponding and apparently full-scale acceptance by the prior art of the use of working enzyme reagents with only a few hours stability.

A significant prior art exception has been the attempt of Mitchel (U.S. Patent No. 3,962,037) to enhance the stability of enzyme-containing reagents for the measurement of glycerol. Mitchell used dextran and Cleland's reagent to achieve satisfactory storage periods of a few days in the glycerol measuring system, relative to the goals of that invention, when the reagent is refrigerated. The means taught by Mitchell differs from the present invention, in addition to other ways, in that it accomplishes only a few day's stability; and it further appears limited to use in a particular assay system (i.e., glycerol).

Also, in the Mitchell patent, the use of a strong reducing agent (Cleland's reagent) apparently eliminates application where NADH is not measured by U.V. absorption but is coupled to a redox dye such as INT, 2-(p-iodophenyl)-3-(p-nitrophenyl)-5-phenyl tetrazolium or redox indicating copper or iron ligands. In contrast, the present invention does permit that special type of NADH quantitation, if desired. Additionally, Cleland's reagent *per se* is a probable contributor to instability of some enzymes.

The present inventive concepts and further contrasts to the prior art

The present invention employs the proper concentrations of a variety of organic and inorganic salts to stabilize important enzymes useful in the measurement of medically important enzymes and other substances in the blood and other body fluids.

Further, with reference to such salts, the present invention uses sodium, potassium, rubidium, ammonium, lithium, or basic amine cations, in combination with organic monophosphate esters (including glycerol monophosphate, ethylene glycol monophosphate), sulfate, certain sulfonated organic acids, carbonate, phosphate, borate, certain dicarboxylic acids, certain dicarboxylic amino acids, or certain monocarboxylic acids, as anions. The concentration of the salt used is generally optimal at a cation concentration of about one equivalent per liter.

It was discovered as a part of the present invention that certain cations, among the several workable cations, are more generally effective than others. From most effective to least effective, they are rubidium, potassium, sodium, ammonium, amine bases, (i.e., TRIS, triethanolamine and imidazole), and lithium ions. Among the anions, sulfate, phosphate esters including alpha or beta-glycerophosphate, ethylene glycol monophosphate, dicarboxylic acids (i.e., malate and succinate), sulfonated organic acids (such as PIPES)

4

and phosphate are best at stabilizing, followed by carbonate and borate, and dicarboxylic amino acids including alanine and aspartic acid, while monocarboxylic acids (i.e., acetate) are least effective.

The differences between all of the anions above are not as great as the differences in effectiveness of cations, except that monocarboxylic acids are far less effective than the other anions. Halogen anions such as chloride and bromide are not workable in the present invention. The diphosphates of ethylene glycol and glycerol are not effective.

In general, the invention is widely applicable to the stabilization of enzymes. The particular cation and anion used and the concentration of the salt formed from particular cation and anion from those found to be effective within this invention, are determined by practical considerations, such as how they affect the rate of the enzyme stabilized or the enzyme being measured.

As an example of choosing salt concentration, hexokinase and glucose 6-phosphate dehydrogenase (G-6-PDH) are stabilized by 0.45 M potassium sulfate; however, the rate of reaction with adenosine triphosphate (ATP) of these two enzymes is markedly diminished. Thus when using these enzymes to measure ATP in a working reagent, or ATP produced by another enzyme in a working reagent (such as in a CPK assay) the concentration of the salt must be diminished as a compromise between the stability of the working reagent and the rate of ATP measurement.

It will be understood, in practicing the invention to stabilize a particular enzyme system, that one should choose a combination of anions and cations taught herein to be workable as a part of this invention which provides the best results in providing a stable useful enzyme reagent.

In certain applications of the present invention, where an anion taught to be workable according to the present invention is a substrate in the enzyme assay for which the working enzyme solution is to be stabilized (i.e., aspartate or alanine in the SGOT or SGPT enzyme assay) the proper cation is chosen from those taught to be useful according to the present invention to complement the carboxylic amino acid anion of the substrate.

Also, combinations of workable anions and cations may be used. A carboxylic acid and sulfate may be used as the anion, and potassium and TRIS may be used as the cation, yielding a mixture of salts known to be useful as a part of the inventive concepts of this invention.

Although carboxylic acids are generally useful, in employing the invention with respect to particular enzyme working reagent systems, it will be apparent to one skilled in the art that a carboxylic acid must have certain properties, such as that it is soluble, does not react with other substrates or ingredients, does not complex metals required for the activity of the enzyme assayed or of the enzyme used as a reagent in an assay in which such complexing would adversely affect the reaction, does not have oxidation or reduction properties incompatible with the reagent system or the enzyme assay, does not have absorbance in the spectral range used in the assay, and does not react with the product of the assay, and is not an inhibitor of enzymes used in the assay or of an enzyme being assayed.

The same consideration as described for the carboxylic acids apply when choosing a particular amine base or sulfonated organic acid when practicing the invention.

Further, one should not choose a combination of anions and cations which include one of the products of the enzyme reaction(s). Thus, malic acid would not be chosen to stabilize the enzyme in a working reagent for SGOT using malic dehydrogenase. Ammonia is not useful in stabilizing reagents for a urea assay using urease and glutamate dehydrogenate because it is an intermediate in the assay. Obviously, ammonia is not included to stabilize glutamate dehydrogenase used to measure ammonia.

The novel concepts of the present invention achieve the heretofore unachieved goal of stabilizing a variety of enzymes in working reagent solutions. This contrasts with the prior art which has not achieved prolonged stability in working reagent solutions but only achieved prolonged stability in stock reagents which were then used to prepare working reagent solutions.

The prior art's use of salt is a further contrast to the present invention. Not only did the prior art achieve only stabilization of stock reagents with salts as opposed to the present ivnention's advantageous stabilization of working reagents, but also there are significant qualitative and quantitative differences in such prior art's use of salts.

The prior art's stabilization of stock solutions used sodium and ammonium sulfate to suspend enzymes which were not generally in solution. By contrast, the present invention teaches the use of a variety of both anions and cations, not merely sodium and ammonium sulfate, to be useful in the stabilization of enzymes in solution in working reagents. The present invention teaches that in many instances there are better or more useful combinations of these anions and cations than ammonium or sodium sulfate. The present invention teaches a general hierarchy among such useful anions and cations which may be combined in various combinations and mixtures. In particular, certain of the cations may be more advantageous in certain applications than sodium or ammonium ions.

Further, the present invention with respect to the stabilization of enzymes in working reagent solutions contrasts quantitatively from the prior art's use of salts to stabilize enzymes in stock suspensions in that the prior art used three to six equivalents per liter of sodium or ammonium cation with a corresponding amount of sulfate anion to stabilize such suspensions. The present invention utilizes cation concentrations of only about one equivalent per liter with corresponding anion concentrations. In several specific embodiments, cation and anion concentrations are far less than one equivalent per liter.

Further contrasting the present invention from the prior art, the present invention has been shown to be useful in combination with novel means of stabilizing NADH or NADPH in enzymatic assays.

Also, the present invention accomplishes the goal of stabilizing working enzyme reagents without the necessity of significantly altering the physical properties of the working enzyme reagents. That is, the working reagents prepared according to the concepts of the present invention contain no ingredients in suspension as opposed to being in solution, need not show significantly altered viscosity, and may be prepared so as to show insignificant differences from water in refractive index. Such unaltered physical properties have significant advantages when such reagents are used in enzyme assays, particularly when used in automated assays.

The present invention is further distinguished over prior art enzyme and NADH stabilization attempts in that it achieves the increasing desire and goal of accommodation to automated procedures. The ever increasing dependence upon laboratory testing necessitates that commonly performed assays be automated, and the most accurate of the methods available for performing these common but vital tests in general employ working reagents containing enzymes.

Unfortunately, however, certain inherent characteristics of automated instrumentation in general use actually magnify the disadvantages of working reagents of very limited stability. Most instrumentation is without means of refrigeration, thus the already short useful life of an enzyme reagent is reduced even further when the assay is performed using an automated procedure which prolongs the time the reagent must remain at warmer temperatures. The problem of costly wastage is also increased due in part to the larger volume of reagent that may not be utilizable due to design limitations of the instrument itself; thus, the more frequently an enzyme reagent must be discarded and replenished with freshly prepared reagent, the more this problem is magnified.

Finally, stabilization efforts which alter the viscosity of a working reagent solution, introduce volatile or corrosive organic solvents, or result in a non-homogeneous mixture render the assay method impractical or even unsuitable at all for automation. Thus the achievements of the present invention accomplish the highly desirous and advantageous goal of allowing the accommodation and adaptation of working reagent solutions of enzymes and NADH to automated procedures.

Stability testing of the concepts and embodiments set forth herein, relating to stability of working reagent solutions of the NADH (or NADPH) and also of the various enzymes, were made both by the actual storage of working reagent solutions thereof for at least one month under refrigeration at 4°C, and/or by the accelerated method required by Lordi and Scott (*J. or Pharm. Sci.*, vol. 54, No. 4, p. 531—537). Certain of the solutions were stability-tested by both methods, for purposes of control and cross-checking, and the accelerated method of Lordi and Scott was shown to be sufficiently reliable, within experimental limits; and the stability was found in all cases, which were tested by only the accelerated method, to be sufficiently prolonged that any differences, between the results given by the accelerated method and by the actual calendar stability term, would be of negligible import in consideration of the one month stability achievement.

Embodiments and test procedures using stabilized working reagents

Subject matter pertaining to NADH/NADPH stabilization is claimed in applicant's co-pending divisional application. However, certain of the embodiments showing NADH stabilization are presented below to enable the understanding of the examples of assay procedures described.

Stabilized NADH I:

A stabilized NADH reagent was prepared by dissolving 0.188 grams of beta nicotinamide adenine dinucleotide, reduced form, disodium salt, dihydrate (NADH) and 25 milligrams of chloramphenicol (an antimicrobial) in approximately 950 ml of deionized water. The pH of this solution was adjusted to 8.3 with approximately 0.2 ml of a solution of 5% TRIS, i.e., 5 grams of TRIS in 100 milliliters of deionized water, and the reagent made to final volume of one liter with deionized water, which made a solution of approximately 80 micromolar TRIS. This solution was filter sterilized, and was determined to be stable for at least one month when stored at 4°C.

Filter sterilization, as referred to herein, was done by drawing the solutions by suction through a Nalge filter with a pore size of 0.22 microns and into a sterile container for storage.

Stabilized NADH II:

A stabilized NADH solution was prepared by dissolving 0.188 grams of NADH, 25 mg of chloramphenicol and 0.03 grams of pyridoxal-5'-phosphate (a co-enzyme for SGOT, used for activating SGOT) in approximately 950 ml of deionized water. The pH of this solution was adjusted to 8.3 with approximately 1 ml of a solution of 5% TRIS, and the final volume was made to one liter with deionized water, which made a solution that was approximately 0.4 millimolar TRIS. This solution was filter sterilized, and was determined to be stable for at least one month when stored at 4°C.

Stabilized NADH III:

A stabilized NADH reagent was prepared by dissolving 0.44 grams of NADH and 25 mg of chloramphenicol in approximately 900 ml of deionized water. The pH of this solution was adjusted to 8.3

**0 072 581**

with 0.8 ml of a 0.1 molar solution of triethanolamine (TEA), and the final volume made to one liter with deionized water. (This calculates to be an 80 micromolar TEA solution.) This solution was filter sterilized and was determined to be stable for at least one month when stored at 4°C.

Stabilized NADH V:

A stabilized NADH reagent was prepared by dissolving 0.298 g of NADH and 25 milligrams of chloramphenicol in approximately 950 ml of deionized water. The pH of this solution was adjusted to 9.2 with approximately 4 ml of 1 M TEA, and the reagent made to final volume of one liter with deionized water. This calculates to be approximately a 4 millimolar TEA solution. This solution was filter sterilized, and was determined to be stable for at least one month when stored at 4°C.

Stabilized NADH VI:

A stabilized NADH reagent was prepared by dissolving 0.328 g of NADH and 25 mg of chloramphenicol in approximately 950 ml of deionized water. The pH of this solution was adjusted to 8.3 with approximately 0.8 ml of 0.1 M TEA. The reagent was made to a final volume of one liter with deionized water and filter sterilized. This calculates to be approximately an 80 micromolar TEA solution. This solution is stable for at least one month when stored at 4°C.

Stabilized LDH enzyme reagent I:

A stabilized LDH enzyme reagent was prepared by dissolving 14.52 g of TRIS, 95 g of alanine, 0.2 g of disodium EDTA (ethylenediaminetetraacetic acid), 20 mg of chloramphenicol, and 1.69 g of alpha ketoglutaric acid, in approximately 800 ml of deionized water. The pH of this solution was adjusted to 7.4 with sulfuric acid, 1200 units of LDH (lactate dehydrogenase) were added, and the final volume was made to one liter with deionized water. The solution was filter sterilized, and was determined to be stable for at least one month when stored at 4°C.

Stabilized LDH enzyme reagent II:

A stabilized LDH enzyme reagent was prepared by dissolving 14.52 g of TRIS, 95 g of alanine, 0.2 g of disodium EDTA, and 20 mg of chloramphenicol in approximately 800 ml of deionized water. The pH of this solution was adjusted to 7.9 with sulfuric acid, 1200 units of LDH were added, and the final volume was made to one liter with deionized water. The solution was filter sterilized, and was determined to be stable for at least one month when stored at 4°C.

Stabilized MDH-LDH enzyme reagent I:

A stabilized enzyme reagent was prepared by dissolving 51.95 grams of potassium hydroxide, 99.9 grams of L-aspartic acid, 36.3 grams of TRIS, 8.1 grams of alpha ketoglutaric acid and 25 mg of chloramphenicol in approximately 750 ml of deionized water. The pH of this solution was adjusted to 7.4 with sulfuric acid. After pH adjustment, 14,000 units of malate dehydrogenase (MDH) and 2,080 units of lactate dehydrogenase (used for destroying pyruvate in the serum to avoid its being mistaken for SGOT activity) were added and the reagent made to volume of one liter with deionized water. This solution was filter sterilized and was determined to be stable for at least one month when stored at 4°C.

In this embodiment, the potassium ions, the aspartate ions, the TRIS ions, and the sulfate ions, provide the stability of the enzymes in this working reagent. The L-aspartic acid and the alpha ketoglutaric acid are provided for the SGOT reaction, and the TRIS is a buffer for the reaction; and thus it is noted that the L-aspartic acid and the TRIS achieve a dual purpose, i.e., that of enzyme stability as well as their role as a substrate in the SGOT reaction itself and as a buffer, respectively.

The inclusion of the potassium, aspartate, TRIS, and sulfate from the substances shown above is equivalent to the addition of a mixture of potassium and TRIS salts of aspartate and sulfate; and the solution contains about 1.2 equivalents of cation.

Stabilized MDH-LDH enzyme reagent II:

A stabilized enzyme reagent was prepared by dissolving 99.9 grams of L-aspartic acid, 8.1 grams of alpha-ketoglutaric acid, 25 mg of chloramphenicol, 36.3 grams of TRIS, and 100 grams of beta-glycerophosphate disodium salt in approximately 700 ml of deionized water. The pH of this solution was adjusted to 7.4 with potassium hydroxide. After pH adjustment, 14,000 units of malate dehydrogenase and 2,080 units of lactate dehydrogenase were added and the reagent brought to a total volume of one liter with deionized water. The solution was filter sterilized, and was determined to be stable for at least one month when stored at 4°C.

In this embodiment, the potassium hydroxide and L-aspartic acid and the beta-glycerophosphate disodium salt provide stability for both the enzymes. The potassium hydroxide serves the dual purpose of adjusting the pH, and providing enzyme stability by its potassium ions; and the L-aspartic acid serves the dual purpose of enzyme stability as well as its role in the SGOT reaction itself.

The inclusion of the aspartate, TRIS, potassium, sodium and glycerophosphate in this way is equivalent to the addition of a mixture of sodium potassium and TRIS salts of aspartate and glycerophosphate; and the solution contains about 1.5 equivalents of cation.

7

**Stabilized MDH-LDH enzyme reagent III:**

A stabilized enzyme reagent was prepared by dissolving 51.95 grams of potassium hydroxide, 99.9 grams of L-aspartic acid, 36.3 grams of TRIS, 8.1 grams of alpha ketoglutaric acid, and 25 mg of chloramphenicol. The pH of this solution was adjusted to 7.4 with the monosodium salt of PIPES (piperazine-N,N′-bis(2-ethanesulfonic acid). After pH adjustment, 14,000 units of MDH and 2,080 units of LDH were added, and the reagent made to volume of one liter with deionized water. This solution was filter sterilized, and was determined to be stable for at least one month when stored at 4°C.

In this embodiment, the sodium ions, the potassium ions, the aspartate ions of the aspartic acid, the TRIS ions, and the PIPES ions provide the stability of the enzymes in this working reagent. The aspartate and alpha ketoglutaric acid are provided for the SGOT reaction, and the TRIS is a buffer for the reaction; and thus it is noted that the L-aspartic acid and the TRIS here achieve a dual purpose, i.e. that of enzyme stability as well as their role in the SGOT reaction itself and their buffering effect.

The inclusion of potassium, sodium, aspartate, TRIS, and PIPES in this way is equivalent to the addition of a mixture of potassium, sodium, and TRIS salts of aspartate and PIPES; and the solution contains about two equivalents of cation.

**Stabilized MDH-LDH enzyme reagent IV:**

This reagent was prepared as in stabilized MDH-LDH Reagent II, but changing the 100 grams of beta-glycerophosphate disodium salt to 100 grams of alpha-glycerophosphate disodium salt. It was observed that this reagent exhibited the same characteristics as the previous MDH-LDH Reagent II with respect to function as well as stability.

**Stabilized MDH-LDH enzyme reagent V:**

This reagent was prepared the same as in stabilized MDH-LDH Reagent II except that the 100 grams of beta-glycerophosphate disodium salt was changed to 100 grams of beta-glycerophosphate dipotassium salt. It was observed that this reagent exhibited the same characteristics as the previous MDH-LDH Reagent II with respect to function as well as stability.

**Stabilized MDH-LDH enzyme reagent VI:**

This reagent was prepared as in stabilized MDH-LDH Reagent II, but changing the 100 grams of beta-glycerophosphate disodium salt to 100 grams of alpha-glycerophosphate dipotassium salt. It was observed that this reagent exhibited the same characteristics as the previous MDH-LDH Reagent II with respect to function as well as stability.

**Stabilized MDH-LDH enzyme reagent VII:**

This reagent was prepared as in stabilized MDH-LDH enzyme reagent II except that the 100 grams of beta-glycerophosphate disodium salt was changed to 80 grams of sodium ethylene glycol monophosphate. It was observed that this reagent exhibited the same characteristics as the previous MDH-LDH reagent II with respect to function as well as stability.

**Stabilized MDH-LDH enzyme reagent VIII:**

This reagent was prepared as in stabilized MDH-LDH enzyme reagent II, but changing the 100 grams of beta-glycerophosphate disodium salt to 80 grams of potassium ethylene glycol monophosphate. It was observed that this reagent exhibited the same characteristics as the previous MDH-LDH Reagent II with respect to function as well as stability.

**Stabilized HK and G-6-PDH enzyme reagent I:**

A stabilized enzyme reagent containing hexkinase (KK) and glucose-6-phosphate dehydrogenase (G-6-PDH) was prepared by dissolving 14.9 grams of triethanolamine (TEA), 100 grams of beta-glycerophosphate disodium salt, 50 mg of chloramphenicol, 5 grams of bovine serum albumin (BSA) (Fraction V), 2.42 grams of adenosine triphosphate (ATP), 1.99 grams of nicotinamide-adenine dinucleotide (NAD), and 0.99 grams of magnesium sulfate heptahydrate. The pH of this solution was adjusted to 7.5 with sulfuric acid. After pH adjustment, 2,000 units of hexokinase and 3,000 units of glucose-6-phosphate dehydrogenase were added. The reagent was made to a final volume of one liter with deionized water and filter sterilized. This solution is stable for at least one month when stored at 4°C.

In this embodiment, the TEA serves the dual function of providing a buffer and stabilizing of the enzymes. Stability of the enzymes is also provided by the beta glycerophosphate disodium salt and the BSA. (With pure raw material enzyme preparations, the inclusion of BSA is unnecessary and of no effect with respect to stability.) The ATP, the NAD, and the magnesium sulfate heptahydrate are used in one of the reactions for the determination of glucose. The sulfuric acid serves the dual role of adjusting pH and providing the sulfate ions which also serve as stabilizers of the enzymes. The inclusion of the TEA and sulfate is equivalent to the addition of a mixture of sodium and TEA salts of glycerophosphate and sulfate, and the solution contains one equivalent of cation.

Stabilized HK and G-6-PDH enzyme reagent II:

A stabilized enzyme reagent containing hexokinase (HK) and glucose-6-phosphate dehydrogenase (G-6-PDH) was prepared by dissolving 37 g of sodium hydroxide, 54 g of succinic acid, 14.9 grams of triethanolamine (TEA), 50 mg of chloramphenicol, 5 grams of bovine serum albumin (BSA), 2.42 grams of adenosine triphosphate (ATP), 1.99 grams of nicotinamide-adenine dinucleotide (NAD), and 0.99 grams of magnesium sulfate heptahydrate. The pH of this solution was adjusted to 7.5 with sulfuric acid. After pH adjustment, 2,000 units of hexokinase and 3,000 units of glucose-6-phosphate dehydrogenase were added, and the reagent was made to a final volume of one liter with deionized water and filter sterilized. This solution is stable for at least one month when stored at 4°C.

In this embodiment, the TEA serves a dual function of providing a buffer as well as stability for the enzymes. The sodium hydroxide and the sulfuric acid provide the sodium ions and the sulfate ions that stabilize the enzymes. Stability of the enzymes is also provided by the BSA. The ATP, the NAD, and the magnesium sulfate heptahydrate are provided for the reaction for the determination of glucose.

The inclusion of sodium, succinate, TEA, and sulfate is equivalent to the addition of a mixture of sodium and TEA salts of succinate and sulfate; and the solution contains about one equivalent of cation.

Stabilized HK and G-6-PDH enzyme reagent III:

This reagent was prepared as in stabilized HK and G-6-PDH Enzyme Reagent II, but changing the 54 grams of succinic acid to 60 grams of malic acid.

Stabilized HK and G-6-PDH enzyme reagent IV:

A stabilized enzyme reagent containing hexokinase (HK) and glucose-6-phosphate dehydrogenate was prepared by dissolving 100 ml of glycerol, 50 mg of chloramphenicol, 2 g of bovine serum albumin (BSA), 50.9 grams of PIPES, 9.6 g of dextrose, 3 g of adenosine monophosphate (AMP), 33.9 grams of creatine phosphate, and 2.66 grams of adenosine diphosphate (ADP) in approximately 700 ml of deionized water. The pH of this solution was adjusted to 6.8 with dilute hydrochloric acid. After pH adjustment, 4,500 units of hexokinase and 6,300 units of glucose-6-phosphate dehydrogenase were added, and the final volume was made to one liter with deionized water. The solution was filter sterilized, and was determined to be stable for at least one month when stored at 4°C.

Here, the PIPES serves as a buffer and also as a stabilizer of the enzymes. Stability of the enzymes is also provided by a synergistic effect of the glycerol and the BSA with the sodium PIPES salt.

The use of salts alone, if used in higher concentration, was found to stabilize the enzymes in the working reagents; however, higher concentrations of salts were found to unduly depress the rate of the CPK reaction for which this working reagent is used. Correspondingly, the use of high concentrations of glycerol was found to stabilize the enzymes in the working reagent; however, such working reagent solutions have high viscosity and other undesirable features of concentrated glycerol solutions. However, the combination of the proper low salt concentration and low glycerol concentration had the effect not achieved by either alone, that is, of stabilizing the working enzyme reagent without unduly depressing the rate of the CPK reaction or suffering other detriments of a high glycerol content; and the working reagents so prepared were otherwise useful in the CPK assay.

The dextrose, the creatine phosphate, the ADP and the enzymes are used in the coupled reaction for the determination of creatine phosphokinase. The AMP serves to inhibit adenylate kinase activity. The inclusion of the monosodium salt of PIPES and creatine phosphate disodium salt in this reagent is equivalent to the addition of a mixture of sodium salts of PIPES and creatine phosphate, and the solution contains 0.35 equivalent of cation.

Stabilized glutamate dehydrogenase (GLDH) enzyme reagent I:

A stabilized glutamate dehydrogenase working reagent was prepared by dissolving 24.2 grams of TRIS, 2.9 grams of alpha ketoglutaric acid, and 52 grams of potassium hydroxide in approximately 600 ml of deionized water. The pH of this solution was adjusted to 8.0 with sulfuric acid and 1800 units of GLDH were added. The reagent was brought to final volume of one liter with deionized water. This solution was filter sterilized, and was determined to be stable for at least one month when stored at 4°C.

In this embodiment, the potassium, the TRIS and the sulfate provide the stability for the enzymes in the working reagent. The TRIS is also the buffer for the reaction. The alpha-ketoglutaric acid is provided for the GLDH reaction.

The inclusion of potassium, TRIS, and sulfate in this way is equivalent to the addition of a mixture of potassium and TRIS salts of sulfate; and the solution contains one equivalent of cation.

Stabilized glutamate dehydrogenase (GLDH) enzyme reagent II:

A stabilized glutamate dehydrogenase working reagent was prepared by dissolving 24.2 grams of TRIS, 2.9 grams of alpha-ketoglutaric acid, 0.3 g dithiothreitol and 52 grams of potassium hydroxide in approximately 600 ml of deionized water. The pH of this solution was adjusted to 8.0 with sulfuric acid and 1800 units of GLDH were added. The reagent was brought to final volume of one liter with deionized water, and was filter sterilized. This reagent is stable for one month when stored at 4°C.

In this embodiment, the potassium, the TRIS and the sulfate provide the stability for the enzymes in the working reagent. The TRIS is also the buffer for the reaction. The alpha-ketoglutaric acid is provided for the GLDH reaction. The inclusion of potassium, TRIS, and sulfate in this way is equivalent to the addition of a mixture of potassium and TRIS salts of sulfate, and the solution contains one equivalent of cation.

Stabilized glutamate dehydrogenase (GLDH) enzyme reagent III:

A stabilized glutamate dehydrogenase working reagent was prepared by dissolving 24.2 grams of TRIS, 2.9 grams of alpha ketoglutaric acid, and 26 grams of potassium hydroxide in approximately 600 ml of deionized water. The pH of this solution was adjusted to 8.0 with sulfuric acid, and 1800 units of GLDH were added. The reagent was brought to final volume of one liter with deionized water, and was filter sterilized. This reagent is stable for one month when stored at 4°C.

In this embodiment, the potassium, the TRIS and the sulfate provide the stability for the enzymes in the working reagent. The TRIS is also the buffer for the reaction. The alpha ketoglutaric acid is provided for the GLDH reaction. The inclusion of potassium, TRIS, and sulfate from the substances shown above is equivalent to the addition of a mixture of potassium and TRIS salts of sulfate; and the solution contains about 0.7 equivalent of cation.

Stabilized urease-glutamate dehydrogenase (GLDH) enzyme reagent:

A stabilized urease-glutamate dehydrogenase (GLDH) working reagent was prepared by dissolving 14.5 grams of TRIS, 4.2 grams of EDTA, 1.2 grams of ADP, 4.96 grams of alpha ketoglutaric acid, and 5 grams of BSA in approximately 500 ml of deionized water. Another solution was prepared by dissolving 52 grams of potassium hydroxide in approximately 300 ml of deionized water and adjusting the pH to 8.0 with sulfuric acid. This solution was added to the first solution and mixed. The pH of the resultant solution was adjusted to 7.5 with sulfuric acid, and 10,000 units of urease and 20,000 units of GLDH were added; and the final volume made to one liter with deionized water. This reagent was filter sterilized, and was determined to be stable for at least one month when stored at 4°C.

In this embodiment, the potassium, TRIS, and sulfate ions provide the stability of the enzymes in the working reagent. The TRIS is also the buffer for the reaction. The ADP is an activator for the GLDH and the BSA is a stabilizer. The alpha-ketoglutaric acid is provided for the GLDH reaction. The inclusion of potassium, TRIS, and sulfate in this way is equivalent to the addition of a mixture of potassium and TRIS salts of sulfate, and the solution contains one equivalent of cation.

Assay procedure for SGOT I:

In performing an SGOT assay, 0.5 ml of several of the stabilized MDH-LDH working reagents I—VIII were separately combined with 2.5 ml of NADH Reagent I. The mixture in each case was warmed to 37°C. Then 200 microliters of serum were added to this mixture and mixed well to assure homogeneity. The solution was transferred to a spectrophotometer, and the change in absorbance at 340 nm was recorded for three minutes. The amount of SGOT in a patient's serum was calculated by use of the following formula:

$$U/liter = \frac{\Delta A/min \times 1000 \times TV \times 1000 \times Tf}{6.22 \times 10^3 \times LP \times SV}$$

where:
$\Delta A/min$ = absorbance change per minute
1000: converts milliliters to liters
TV = total reaction volume or 3.2 ml
1000: converts millimoles to micromoles
Tf = temperature conversion factor (1.0 at 37°C)
$6.22 \times 10^3$ = molar extinction coefficient of NADH (340 nm)
LP = light path in centimeters
SV = sample volume (0.20 ml)
U = International Units of enzyme activity, defined as the amount of enzyme which converts one micromole of substrate in one minute at standard conditions.

Alternatively, the amount of SGOT in a patient's serum could be calculated from a known standard that was assayed in the manner just described for a patient. The relationship between the standard and the patient is expressed as follows:

$$U \text{ of SGOT in known standard} \times \frac{\Delta A \text{ patient}}{\Delta A \text{ standard}} = U \text{ of SGOT in patient's serum}$$

Assay procedure for SGOT II:

In another application using any of stabilized MDH-LDH Reagents I—VIII and stabilized NADH reagent II, 200 microliters of a patient's serum was added to 2.5 ml of the stabilized NADH reagent, and allowed to incubate at 37°C for 5 minutes. Then 0.5 ml of prewarmed stabilized MDH-LDH enzyme reagent (prewarmed to 37°C) was added and the resultant mixture thoroughly mixed to insure homogeneity. The reaction mixture was then transferred to a spectrophotometer and the loss of absorbance at 340 nm (by the oxidation of NADH to NAD) was measured for a period of three minutes. The amount of SGOT in patient's serum was calculated from the formula using the molar extinction coefficient as the previous example or from the calculation involving a known standard as in the previous example.

This procedure, using the stabilized NADH reagent referred to herein as NADH Reagent II, containing pyridoxal-5'-phosphate, is different from the previous embodiment in that an incubation period is needed for the saturation of the SGOT with the pyridoxal-5'-phosphate not used in the NADH Reagent I of the previous SGOT assay.

Assay procedure for SGPT:

For the SGPT assay, 1.0 ml of stabilized NADH Reagent III was combined with 2.0 ml of stabilized LDH enzyme reagent, and the mixture warmed to 37°C. Then 200 microliters of serum were added to this mixture, and the solution was mixed to assure homogeneity. The solution was then transferred to a spectrophotometer and the absorbance change at 340 nm was recorded. The amount of SGPT in the serum was calculated from the formula involving change in absorbance per minute, as in SGOT Assay I, or from the calculation involving a known standard as in the SGOT Assay I.

Assay procedure for glucose:

For assaying for glucose, 20 microliters of serum or standard was added to 3.0 ml of stabilized HK and G-6-PDH Reagent I, II, or III. which had been prewarmed to 37°C, and mixed. The reagent and serum were incubated together for 5 minutes at 37°C. This mixture was then transferred to a spectrophotometer that had had the optical density (O.D.) adjusted to read zero at 340 nm when 3.0 ml of stabilized hexokinase working reagent containing 20 microliters of 0.85% sodium chloride was read, and the O.D. of the solution containing serum was then read. The amount of glucose in the serum can be calculated from this absorbance and the absorbance of a standard that had been read according to the following equation:

$$\text{Glucose concentration of standard} \times \frac{\text{O.D. serum}}{\text{O.D. standard}} = \text{glucose concentration of serum}$$

Assay procedure for CPK I:

In this assay, 1.0 ml of stabilized HK and G-6-PDH Enzyme Reagent IV was combined with 2.0 ml of stabilized Coenzyme Sulfhydryl Reagent disclosed in the co-pending divisional application number 79103451.5 and warmed to 37°C. Then 100 microliters of serum or standard was added to the prewarmed mixture, and allowed to incubate for two minutes, then transferred to a spectrophotometer; and the change in absorbance at 340 nm was recorded for 3 to 5 minutes above a reagent blank. The change in absorbance per minute was calculated, and the units of CPK activity were calculated by using the standard's change in absorbance, in the following equation:

$$\text{Units CPK in standard} \times \frac{\Delta A \text{ serum}}{\Delta A \text{ standard}} = \text{units CPK in patient's serum}$$

Assay procedure for serum ammonia:

In performing the serum ammonia assay, samples must first be deproteinated by the following procedure: Cool samples in an ice bath, and deproteinize with an equal volume of trichloroacetic acid solution (10 g trichloroacetic acid in 100 ml of deionized water). After 5 to 10 minutes centrifuge off the precipitated protein and neutralize the supernatant fluid with potassium bicarbonate solution (20 g $KHCO_3$ in 100 ml of deionized water).

To perform the ammonia assay, 1.5 ml of the stabilized NADH Reagent V was combined with 1.5 ml of stabilized Glutamate Dehydrogenase Enzyme Reagent I (II and III were also found to function), and the mixture warmed to 37°C. After warming, 200 microliters of deproteinated serum were added. The mixture was transferred to a spectrophotometer, and the change in absorbance per minute at 340 nm was recorded. The amount of ammonia in a patient's serum was calculated from the equation as in the assay procedure for SGOT.

Assay procedure for blood urea nitrogen (BUN):

To perform the BUN assay, 1.5 ml of the stabilized NADH Reagent VI was combined with 1.5 ml of the stabilized urease-glutamate dehydrogenase working reagent and warmed to 37°C. After warming, 20 microliters of serum were added to the mixture, and the mixture was transferred to a spectrophotometer where the change in absorbance per minute at 340 nm was recorded. A BUN standard was also run in the procedure just described, and the concentration of BUN in the patient's serum was calculated from this standard according to the following equation:

11

$$\text{Urea nitrogen} = \Delta A/\text{min} \times S \times D$$

where:

$\Delta A$ = change in absorbance at 340 nm

S = calculation factor derived from the standard (see below)

D = dilution factor

$$S = \frac{\text{concentration of the standard}}{A/\text{min of the standard}}$$

Accordingly, it will thus be seen from the foregoing description of the invention according to the embodiments of the invention herein set forth, that the present invention provides a new and useful means for the highly advantageous stabilization of working solutions of NADH, NADPH, and enzymes, in their aqueous working reagent use in several assays and procedures illustrative of and embodying the inventive concepts. The objects and advantages of the invention are thus accomplished and achieved, and the details of the concepts are illustrated in the various embodiments.

It will be understood that certain modifications and variations of the specific and general concepts of the invention may be effected without departing from the concepts heretofore described; accordingly, the invention is not to be considered limited to the specific form or embodiments set forth herein for the purpose of disclosing and illustrating the inventive concepts discovered and herein applied.

For example, there is a wide variety of antimicrobial agents which may be advantageously used in practicing the present invention, and are of course desirable to keep microbial growth out of reagents; provided, however, that they are such that the action of the enzymes is not inhibited, nor do the antimicrobials have unusually high absorbance levels at the wavelength at which spectrophotometric measurements are to be made, nor do they react with an ingredient of the assay.

Further, it is of course desirable to use raw material enzymes for inclusion in the stabilized enzyme working solutions which are desirably free or relatively free from proteolytic enzymes, for it is well known that proteolytic enzymes tend to destroy working reagent enzymes when in solution therewith.

## Claims

1. A process for prolonging the stable life of aqueous working reagent solutions of enzymes which are directly or indirectly detected by the oxidation of NADH or NADPH or the reduction of NAD or NADP, comprising the inclusion in said solution of one or more cations from the group consisting of sodium, potassium, rubidium, ammonium, lithium, TRIS, and triethanolamine, and one or more anions from the group consisting of ethylene glycol monophosphate, glycerol monophosphate, carbonate, phosphate, borate, sulfate, succinate, malate, aspartate, glycerophosphate, alanine, and PIPES, the concentration of said cations or anions being between about 0.2 and 2.8 equivalents per liter of the working reagent solution, the pH of said solution being about 8.0 or less, and the solution being free of stabilizing amounts of glycerol and BSA.

2. The process of claim 1, wherein the enzymes are MDH and/or LDH, and wherein the cations are from the group of sodium, potassium, and TRIS, and the anions are from the group of aspartate, sulfate, glycerophosphate, PIPES, alanine, and ethylene glycol monophosphate.

3. The process of claim 1, wherein the enzymes are HK and G-6-PDH, and wherein the cations are from the group of sodium and triethanolamine and the anions are from the group of sulfate, glycerophosphate, succinate, malate, and PIPES.

4. The process of claim 1, wherein the enzyme is GLDH, and wherein the cations are potassium and TRIS and the anion is sulfate.

5. The process of claim 1, wherein the enzymes are urease and GLDH, and wherein the cations are potassium and TRIS and the anion is sulfate.

6. The process of claim 1, wherein the enzyme is LDH, and wherein the cations are from the group of sodium, potassium, and TRIS, and the anions are from the group of aspartate, sulfate, glycerophosphate, PIPES, alanine, and ethylene glycol monophosphate.

7. An aqueous working reagent solution stabilized according to the process of claim 1.

8. An aquoeus working reagent solution stabilized according to the process of claim 2 for use in an assay for SGOT.

9. An aqueous working reagent solution stabilized according to the process of claim 3 for use in an assay for glucose.

10. An aqueous working reagent solution stabilized according to the process of claim 3 for use in an assay for CPK.

11. An aqueous working reagent solution stabilized according to the process of claim 4 for use in an assay for ammonia.

12. An aqueous working reagent solution stabilized according to the process of claim 5 for use in an assay for urea.

13. An aqueous working reagent solution stabilized according to the process of claim 6 for use in an assay for SGPT.

**Patentansprüche**

1. Verfahren zur Verlängerung der Lebensdauer von wässrigen Arbeitsreagenz-Lösungen von Enzymen, die direkt oder indirekt durch Oxidation von NADH oder durch Reduktion von NAD oder NADP nachgewiesen werden, dadurch gekennzeichnet, daß in die Lösung ein oder mehrere Kationen aus der Gruppe bestehend aus Natrium, Kalium, Rubidium, Ammonium, Lithium, TRIS und Triäthanolamin, und ein oder mehrere Anionen aus der Gruppe bestehend aus Äthylenglykolmonophosphat, Glycerinmonophosphat, Carbonaten, Phosphaten, Boraten, Sulfaten, Succinaten, Malaten, Aspartaten, Glycerinphosphat, Alanin und PIPES einbezogen werden, wobei die Konzentration dieser Kationen und Anionen zwischen etwa 0.2 und 2.8 Äquivalenten pro Liter Arbeitsreagenz-Lösung und der $p_H$-Wert dieser Lösung etwa 8.0 oder darunter beträgt und die Lösung frei von stabilisierenden Mengen an Glycerin und BSA ist.

2. Verfahren nach Anspruch 1, dadurch gekennzeichnet, daß die Enzyme MDH und/oder LDH sind und daß die Kationen Natrium, Kalium oder TRIS und die Anionen Aspartate, Sulfate, Glycerinphosphat, PIPES, Alanin oder Äthylenglykolmonophosphat sind.

3. Verfahren nach Anspruch 1, dadurch gekennzeichnet, daß die Enzyme HK und G-6-PDH sind und daß die Kationen Natrium oder Triäthanolamin und die Anionen Sulfate, Glycerinphosphat, Succinate, Malate oder PIPES sind.

4. Verfahren nach Anspruch 1, dadurch gekennzeichnet, daß das Enzym GLDH ist und daß die Kationen Kalium oder TRIS sind und das Anion ein Sulfat ist.

5. Verfahren nach Anspruch 1, dadurch gekennzeichnet, daß die Enzyme Urease und GLDH sind und daß die Kationen Kalium oder TRIS sind und das Anion ein Sulfat ist.

6. Verfahren nach Anspruch 1, dadurch gekennzeichnet, daß das Enzym LDH ist und daß die Kationen Natrium, Kalium oder TRIS und die Anionen Aspartate, Sulfate, Glycerinphosphat, PIPES, Alanin oder Äthylenglykolmonophosphat sind.

7. Wässrige Arbeitsreagenz-Lösung, stabilisiert nach dem Verfahren gemäß Anspruch 1.

8. Wässrige Arbeitsreagenz-Lösung, stabilisiert nach dem Verfahren nach Anspruch 2 zur Verwendung bei der Untersuchung auf SGOT.

9. Wässrige Arbeitsreagenz-Lösung, stabilisiert nach dem verfahren gemäß Anspruch 3 zur Verwendung bei der Untersuchung auf Glukose.

10. Wässrige Arbeitsreagenz-Lösung, stabilisiert nach dem Verfahren gemäß Anspruch 3 zur Verwendung bei der Untersuchung auf CPK.

11. Wässrige Arbeitsreagenz-Lösung, stabilisiert nach dem Verfahren gemäß Anspruch 4 zur Verwendung bei der Untersuchung auf Ammoniak.

12. Wässrige · Arbeitsreagenz-Lösung, stabilisiert nach dem Verfahren gemäß Anspruch 5 zur Verwendung bei der Untersuchung auf Harnstoff.

13. Wässrige Arbeitsreagenz-Lösung, stabilisiert nach dem Verfahren gemäß Anspruch 6 zur Verwendung bei der Untersuchung auf SGPT.

**Revendications**

1. Procédé visant à prolonger la durée de vie de solutions aqueuses réactives de travail d'enzymes qui sont décelés directement ou indirectement par l'oxydation de NAOH ou par la réduction de NAD ou NADP, caractérisé par le fait qu'un ou plusieurs cations du groupe formé de sodium, potassium, rubidium, ammonium, lithium, TRIS et triéthanolamine ainsi qu'un ou plusieurs anions du groupe formé de monophosphate d'éthylène glycol, monophosphate de glycérine, carbonates, phosphates, borates, sulfates, succinates, malates, aspartates, phosphate de glycérine, alanine et PIPES sont inclus dans la solution, la concentration de ces cations et anions étant comprise entre env. 0.2 et 2.8 équivalents par litre de solution réactive de travail, le pH de cette solution étant d'env. 8.0 ou inférieur à cette valeur et la solution étant exempte de quantités stabilisatrices de glycérine et BSA.

2. Procédé selon la revendication 1, caractérisé, d'une part, par le fait que les enzymes sont des MDH et/ou LDH et, d'autre part, par le fait que les cations sont du sodium, potassium ou TRIS et les anions des aspartates, sulfates, du phosphate de glycérine, du PIPES, de l'alanine ou du monophosphate d'éthylène glycol.

3. Procédé selon la revendication 1, caractérisé, d'une part, par le fait que les enzymes sont des HK et G-6-PDH et, d'autre part, par le fait que les cations sont du sodium ou du triéthanolamine et les anions des sulfates, du phosphate de glycérine, des succinates, des malates ou du PIPES.

4. Procédé selon la revendication 1, caractérisé, d'une part, par le fait que l'enzyme est du GLDH et, d'autre part, par le fait que les cations sont du potassium ou du TRIS et l'anion est un sulfate.

5. Procédé selon la revendication 1, caractérisé, d'un part, par le fait que les enzymes sont de l'uréase et du GLDH et, d'autre part, par le fait que les cations sont du potassium ou du TRIS et l'anion est un sulfate.

6. Procédé selon la revendication 1, caractérisé, d'une part, par le fait que l'enzyme est du LDH et, d'autre part, par le fait que les cations sont du sodium, du potassium ou du TRIS et les anions des

**0 072 581**

aspartates, des sulfates, du phosphate de glycérine, du PIPES, de l'alanine ou du monophosphate d'éthylène glycol.

7. Solution aqueuse réactive de travail, stabilisée suivant le procédé de la revendication 1.

8. Solution aqueuse réactive de travail, stabilisée suivant le procédé de la revendication 2, à utiliser pour la recherche de SGOT.

9. Solution aqueuse réactive de travail, stabilisée suivant le procédé de la revendication 3, à utiliser pour la recherche de glucose.

10. Solution aqueuse réactive de travail, stabilisée suivant le procédé de la revendication 3, à utiliser pour la recherche de CPK.

11. Solution aqueuse réactive de travail, stabilisée suivant le procédé de la revendication 4, à utiliser pour la recherche d'ammoniac.

12. Solution aqueuse réactive de travail, stabilisée suivant le procédé de la revendication 5, à utiliser pour la recherche d'urée.

13. Solution aqueuse réactive de travail, stabilisée suivant le procédé de la revendication 6, à utiliser pour la recherche de SGPT.

14